# EUROPEAN PATENT APPLICATION

(11) **EP 1 402 950 A1**
(43) Date of publication of application: **31.03.2004**
(21) Application number: 03020669.2
(22) Date of filing: 11.09.2003
(51) Int. Cl.: B01J 31/02, B01J 31/04, B01J 35/12, C07C 5/27

(54) **Catalyst and process of paraffin hydrocarbon conversion**

(30) Priority: 25.09.2002 DK 200201415
(71) Applicant: Haldor Topsoe A/S, 2800 Kgs. Lyngby (DK)
(72) Inventor: Zavilla, John, 2980 Kokkedal (DK); Herbst, Konrad, 2800 Kgs. Lyngby (DK); Houzvicka, Jindrich, 51101 Turnov (CZ); Hommeltoft, Sven Ivar, 3400 Hillerod (DK)

(57) **Abstract**

A catalyst composition and process for the conversion of linear and/or branched paraffin hydrocarbons based on the use of an ionic liquid catalyst in combination with a Brønsted Acid, which provides a catalytic composition with an increased activity compared with said ionic liquid. Under suitable reaction conditions this conversion is leading to paraffin hydrocarbon fraction with higher octane number.

## Description

### BACKGROUND OF THE INVENTION AND PRIOR ART

The present invention relates to a process for the conversion of paraffin hydrocarbons catalysed by a mixture of an acidic ionic liquid catalyst and a Brønsted acid (proton donating acid).

Paraffin hydrocarbons with high degree of branching are known to be useful blending components for motor gasoline due to their high octane numbers. Such paraffin hydrocarbon fraction can be produced in an isomerisation process increasing the octane number of the C₄-C₉ cuts. Isomerisation of C₄, C₅ and C₆ paraffins are common refinery processes based on use of e.g. an acidic Friedel-Crafts catalyst such as AlCl₃. Processes including higher fractions (C₇ to C₉ hydrocarbons) meet with significant difficulties due to low selectivity and low octane number of the once-through products.

A relatively new class of acidic catalysts based on ionic liquids, e.g. produced from AlCl₃, has recently been described in the literature (P. Wasserscheid, W. Keim, Angew. Chem., Int. Ed., 2000, V. 39, pages 3772-3789; T. Welton, Chem. Rev., 1999, V. 99, pages 2071-2083). This group of compounds also referred to as molten salts are constituted of:
(1) an inorganic anion typically formed from metal halides, such as AlCl₄⁻, Al₂Cl₇⁻ or other inorganic anions (SO₄²⁻ , NO₃⁻, PF₆⁻, CF₃SO₃⁻, BF₄⁻ etc.), and
(2) an organic cation typically derived from N-heterocyclic or alkylammonium entities.

The melting point of ionic liquids is relatively low and an increasing number of ionic liquids are described with melting points below room temperature. Below some characteristics of ionic liquids are listed:
(1) They have a liquid range of about 300°C.
(2) They are good solvents for a wide range of inorganic, organic and polymeric materials.
(3) They exhibit Brønsted and Lewis acidity as well as superacidity.
(4) They have low or no vapour pressure.
(5) Most ionic liquids are thermally stable up to near 200°C, some ionic liquids are stable at much higher temperature (about 400-450°C).
(6) They are relatively cheap and easy to prepare and upscale.
(7) They are non-flammable and easy in operation.
(8) They are highly polar but non-coordinating materials.

Thus, the term "ionic liquid" in the following description shall refer to salts consisting of ions, which exist in the melted form and consist of organic nitrogen-containing heterocyclic or aliphatic cations and inorganic anions.

Ionic liquids most frequently demonstrate Lewis acidic properties once they are formed by metal halides. In many cases, however, the ionic liquids show also strong Brønsted (proton) acidity. The proton acidity may originate both from the cation if it contains a proton at the quarternized N atom or from the anion if it contains protons for instance in HSO₄⁻, H₂PO₄⁻.

Also HCl produced via partial hydrolysis for example of the chloroaluminate anion can explain strong proton acidity of the ionic liquids. Addition of a Brønsted Acid, e.g. H₂SO₄, to an ionic liquid containing chloroaluminate anions, will also increase the amount of protons in the medium and in case the Brønsted Acid react with the ionic liquid HCl is liberated to the medium.

Lewis-acidic properties of ionic liquids are governed by two major factors: (1) the nature of the anion, and (2) the molar ratio of the organic part to the inorganic part (for instance in the case of ionic liquids based on metal halides Me (Hal)ₙ by the molar fraction of Me (Hal)ₙ). If X_{Me(Hal)n}<0.5 the ionic liquid is called basic; if X_{Me(Hal)n}=0.5 this is the case of neutral ionic liquid, and finally if X_{Me(Hal)n}>0.5 the ionic liquid can be classified as acidic or in some cases superacidic.

The effect of superacidity of ionic liquids is quite frequently observed for AlCl₃-based compositions. Sometimes this effect is related to the presence of dry HCl in the system, which is dissolved in the ionic liquid. The Hammett function H₀ for such systems (H₀=-18) indicates superacidic properties of the ionic liquids comparable with those of HF-TaF₅ (H₀=-16) and "magic acid" HF-SbF₅ or FSO₃H-SbF₅ (H₀=-25). All these systems are much stronger acids as compared to the conventional 100% H₂SO₄ (H₀=-12), which marks the border of superacidity. Such ionic liquids are also stronger than the solid superacids like SO₄/ZrO₂ (H₀=-16), H₃PW₁₂O₄₀ (H₀=-13.5) or H-Nafion (H₀=-12).

### SUMMARY OF THE INVENTION

The object of the present invention is to provide an improved catalyst and a process for the conversion of linear and/or branched paraffin hydrocarbons.

Based on the observation that ionic liquid catalyst combined with a Brønsted Acid provides a catalytic composition with improved activity compared to ionic liquid this invention is a catalyst composition for use in a hydrocarbon conversion process with the provision that the hydrocarbon conversion process is not cracking of polymers, which composition comprises
(a) an ionic liquid catalyst comprised of a N-containing heterocyclic and/or aliphatic organic cation and an inorganic anion derived from metal halides or mixed metal halides, and
(b) one or more Brønsted Acids .

It has been found that the above catalyst composition is particularly useful in isomerisation of paraffin hydrocarbons.

Consequently, a further aspect of the invention is a process for isomerisation of hydrocarbon feed comprising paraffinic hydrocarbons in the presence of a composite catalyst comprising
(a) an ionic liquid catalyst comprised of a N-containing heterocyclic and/or aliphatic organic cation and an inorganic anion derived from metal halides or mixed metal halides, and
(b) one or more Brønsted Acids.

### DETAILED DESCRIPTION OF THE INVENTION

The ionic liquids used for preparation of the catalyst composition and the hydrocarbon isomerisation reaction represent salts formed by an organic cation such as N-containing heterocyclic or N-containing aliphatic moiety and an inorganic anion, which may be an anion derived from metal halides or mixed metal halides. The cation may be an alkyl substituted pyridinium, piperidinium, quinolinium (or similar amine compounds) with one or several alkyl or aryl groups or an alkyl ammonium (mono-alkyl, di-alkyl, trialkyl or tetra-alkyl ammonium compound). The anion may be derived from any metal halide with strong Lewis acidic properties for instance AlCl₄⁻, AlBr₄⁻, GaCl₄⁻, Al₂Cl₇⁻, Al₂Cl₆Br⁻ and the like. The ionic liquid chosen for paraffin isomerisation may be characterised by the amine: Lewis acid molar ratio from 1:3 to 2:1, more preferably from 1:2.5 to 1:1.

The Brønsted Acid used in combination with the ionic liquids as catalysts can be chosen from HCl, HBr, CH₃SO₃H (and other alkane sulphonic acids), CH₃CO₂H (and other carboxylic acids), CF₃SO₃H (and other fluorinated alkane sulphonic acids), CF₃CO₂H (and other fluorinated carboxylic acids), ClSO₃H, FSO₃H, H₂SO₄, H₃PO₄ and the like. Physical mixtures of several of these compounds may also be used.

The Brønsted Acid can be added in gaseous, liquid or solid form to the ionic liquid in some cases resulting in the formation of a heterogeneous mixture. Some of the Brønsted Acids react with the ionic liquid liberating HCl (if the ionic liquid is based on e.g. a chloroaluminate compound).

The mixture of ionic liquid and Brønsted Acid can be used as catalyst as such, or it can be treated by appropriate means, e.g. heat treatment.

The catalyst composition according to the invention gives a novel strongly acidic catalyst, which is significantly more active than common ionic liquids. As such it can be used in a large number of hydrocarbon conversions, where also room-temperature ionic liquids are used. Among these processes of potential commercial interest are various alkylation, oligomerisation and isomerisation reactions. The list of such possible applications is given in D. Zhao, M. Wu, Y. Kou, E. Min, Catalysis Today, V. 74, 2002, pages 157-189, whose content hereby is incorporated into this patent disclosure by reference thereto.

The solubility of hydrocarbons in ionic liquids is limited and for instance paraffins and naphthenes are generally immiscible with ionic liquids. Olefins and aromatic compounds demonstrate a clear dependence of the solubility on the oleophilic properties of the ionic liquid. The longer the chain length of the radical attached to the N-heterocyclic. moiety, the higher the solubility of olefins and aromatics in the ionic liquids. However, most of the commonly used organic solvents and reagents are immiscible with ionic liquids. This simplifies the use of ionic liquids in a biphasic system and provides a procedure for a simple product/catalyst separation.

Paraffin isomerisation can be carried out in pressurised equipment under high pressure or in a glass vessel at atmospheric pressure. The pressure in the autoclave can be varied from 1 bar to 60 bar. Any gas like helium, argon, nitrogen, hydrogen or dry air can be used in the reaction. The reaction temperature can vary in a range from -30°C to 150°C. Temperatures out of this range can also be used although they are less preferred.

Linear n-paraffins such as n-butane, n-pentane, n-hexane, n-heptane, n-octane, n-nonane and monomethylalkanes such as 2-methylhexane and 3-methylhexane or a mixture thereof can be used as substrates of the isomerisation process forming a product containing paraffin hydrocarbons with a higher degree of branching.

The hydrocarbon feeds used for the isomerisation experiments in this disclosure is specified below.

### Experimental Procedures 1-3:

17.7 wt% n-heptane, 21.0 wt% 2-methylhexane, 20.9 wt% 3-methylhexane, 36.7 wt% methylcyclohexane, 1.1 wt% 2,4-dimethylpentane, 1,6 wt% 2,3 dimethylpentane and 1.0 wt% of other C7 isomer compounds.

### Experimental Procedure 4:

19.5 wt% n-heptane, 20.4 wt% 2-methylhexane, 20 wt% 3-methylhexane, 35.6 wt% methylcyclohexane, 1 wt% 2,4-dimethylpentane, 1,5 wt% 2,3 dimethylpentane and 2.0 wt% of other C7 isomer compounds.

### EXAMPLES

### Example 1

In an inert atmosphere (N₂), trimethylamine hydrochloride (39.13 g, 0.409 mole) is added to aluminium chloride (98.28 g, 0.737 mole). The light-brown viscous melt, which forms are heated to 90°C under stirring and kept at this temperature for 2 hours. From the resulting liquid may precipitate some solid AlCl₃ after cooling to room temperature. In the isomerisation experiments described below only the liquid phase has been used as catalyst. The ionic liquid can be stored in inert atmosphere (N₂) without decomposition.

### Example 2

In an inert atmosphere (N₂), a 2-neck Schlenk flask equipped with a mechanical stirrer is charged with 30 ml ionic liquid (42 g) prepared according to Example 1 and 30 ml of the organic hydrocarbon feed. A certain amount of Brønsted Acid (see Table 1) is added to the mixture. The system is vigorously stirred (700 rpm) at constant temperature. Samples of the hydrocarbon phase are taken at regular intervals and analyzed by a gas chromatograph.

### Example 3

In an inert atmosphere (N₂) a 2-neck Schlenk flask is charged with 30 ml ionic liquid (42 g) prepared according to Example 1 and a certain amount of Brønsted Acid (see Table 1). This mixture is heated to 90°C and left under stirring for 1 hour. After cooling to room temperature, 30 ml of the organic hydrocarbon feed is added to the mixture. The system is vigorously stirred (700 rpm) using mechanical agitation at constant temperature. Samples of the hydrocarbon phase are taken at regular intervals and analyzed by a gas chromatograph.

### Example 4

In an inert atmosphere (N₂), an autoclave with mechanical stirrer is charged with 40 ml ionic liquid (56 g) prepared according to Example 1 and 40 ml of the organic hydrocarbon feed. A certain amount of Brønsted acid (see Table 1) is added to the mixture. The system is pressurised with 5 bar helium (for sampling) and afterwards vigorously stirred (700 rpm) at constant temperature. Samples of the hydrocarbon phase are taken at regular intervals and analysed by a gas chromatograph.

### Example 5

In an inert atmosphere (N2), a 2-neck Schlenk flask equipped with a mechanical stirrer is charged with 30 ml ionic liquid (42 g) prepared according to Example 1. A stream of HCl gas is bobbled through the ionic liquid for 30 min, thereby dissolving HCl in the ionic liquid. 30 ml of the organic hydrocarbon feed, which earlier has been saturated with HCl gas, are added to the ionic liquid. The system is vigorously stirred (700 rpm) at constant temperature. Samples of the hydrocarbon phase are taken at regular intervals and analyzed by a gas chromatograph.

**Table 1**

| **DEFINITIONS** | | | | | | |
|---|---|---|---|---|---|---|
| Multi-branched C₇ products: Dimethylpentanes and trimethylbutane. | | | | | | |
| C₆- products: Compounds containing six and less than six carbon atoms. | | | | | | |
| C₈₊ products: Compounds containing eight and more than eight carbon atoms. | | | | | | |
| Normalised yield of multi-branched C₇ products is defined as: 100x(sum of multi-branched C₇ products)/(sum of C₇ compounds excluding cyclic C₇ compounds). | | | | | | |
| Selectivity to multi-branched C₇ products is defined as: 100x(sum of multi-branched C₇ products)/(sum of multi-branched C₇ products + C₆- products + C₈₊ products). | | | | | | |

| Example | Brønsted acid | Amount of Brønsted acid (g) | Temperature (°C) | Time (min) | Normalised yield of multi-branched isomers (wt%) | Selectivity (wt%) |
|---|---|---|---|---|---|---|
| 2 (a) | None (reference example) | | 25 | 30 | 6.6 | 97.8 |
| | | | | 60 | 7.6 | 98.2 |
| | | | | 90 | 8.2 | 98.6 |
| | | | | 120 | 8.6 | 98.6 |
| | | | | 180 | 9.5 | 99.1 |
| | | | | 240 | 10.2 | 99.3 |
| | | | | 300 | 10.7 | 99.2 |
| 2 (b) | H₂SO₄ (96 wt-%) | 2.30 | 25 | 30 | 7.2 | 98.1 |
| | | | | 60 | 11.9 | 98.7 |
| | | | | 90 | 17.7 | 98.4 |
| | | | | 120 | 24.5 | 96.6 |
| | | | | 150 | 28.2 | 93.9 |
| | | | | 180 | 29.5 | 91.2 |
| 2 (c) | H₂SO₄ (96 wt-%) | 5.52 | 25 | 5 | 5.3 | 80.8 |
| | | | | 10 | 6.6 | 98.4 |
| | | | | 15 | 8.1 | 98.7 |
| | | | | 30 | 11.7 | 99.1 |
| | | | | 60 | 18.3 | 98.6 |
| 2 (d) | H₂SO₄ (96 wt-%) | 6.81 | 25 | 30 | 8.4 | 98.2 |
| | | | | 60 | 15.5 | 98.0 |
| | | | | 90 | 19.8 | 92.7 |
| | | | | 120 | 27.0 | 90.3 |
| | | | | 150 | 28.3 | 90.4 |
| | | | | 180 | 28.6 | 89.2 |
| 2 (e) | CF₃SO₃H | 3.48 | 25 | 5 | 5.9 | 96.8 |
| | | | | 10 | 7.3 | 98.6 |
| | | | | 15 | 8.8 | 98.8 |
| | | | | 30 | 11.6 | 99.0 |
| | | | | 60 | 14.6 | 99.1 |
| | | | | 150 | 16.9 | 99.0 |
| | | | | 180 | 17.3 | 99.1 |
| | | | | 240 | 18.0 | 99.0 |
| 2 (f) | CF₃SO₃H | 6.78 | 25 | 30 | 9.2 | 98.6 |
| | | | | 60 | 14.3 | 98.8 |
| | | | | 90 | 17.2 | 98.3 |
| | | | | 120 | 19.5 | 98.3 |
| | | | | 150 | 20.0 | 98.1 |
| | | | | 180 | 20.4 | 98.2 |
| 2 (g) | CF₃SO₃H | 10.18 | 25 | 30 | 7.0 | 98.5 |
| | | | | 60 | 7.9 | 98.8 |
| | | | | 90 | 8.2 | 99.0 |
| | | | | 120 | 8.5 | 99.0 |
| | | | | 150 | 8.7 | 98.8 |
| | | | | 180 | 8.9 | 97.0 |
| 2 (h) | ClSO₃H | 0.53 | 0 | 30 | 5.3 | 97.8 |
| | | | | 60 | 6.3 | 98.4 |
| | | | | 90 | 7.4 | 98.8 |
| | | | | 120 | 8.8 | 98.7 |
| | | | | 150 | 10.8 | 98.2 |
| | | | | 180 | 13.7 | 99.3 |
| 2 (i) | ClSO₃H | 1.40 | 25 | 30 | 26.4 | 90.0 |
| | | | | 60 | 34.5 | 72.0 |
| | | | | 90 | 35.9 | 69.6 |
| | | | | 120 | 36.3 | 68.1 |
| | | | | 150 | 36.4 | 68.2 |
| | | | | 180 | 36.3 | 66.4 |
| 2 (j) | ClSO₃H | 2.72 | 25 | 5 | 9.2 | 97.9 |
| | | | | 10 | 16.3 | 96.2 |
| | | | | 15 | 23.0 | 92.4 |
| | | | | 30 | 33.3 | 76.8 |
| | | | | 60 | 37.8 | 66.1 |
| | | | | 120 | 38.7 | 64.0 |
| | | | | 180 | 38.5 | 62.5 |
| 2 (k) | H₃PO₄ | 2.27 | 25 | 30 | 7.5 | 98.0 |
| | | | | 60 | 10.9 | 98.7 |
| | | | | 90 | 12.5 | 97.3 |
| | | | | 120 | 13.2 | 98.6 |
| | | | | 150 | 13.7 | 98.5 |
| | | | | 180 | 14.0 | 98.7 |
| 2 (l) | H₃PO₄ | 4.54 | 25 | 30 | 8.1 | 97.7 |
| | | | | 60 | 11.2 | 97.4 |
| | | | | 90 | 12.7 | 97.9 |
| | | | | 120 | 13.4 | 97.8 |
| | | | | 150 | 13.8 | 99.0 |
| | | | | 180 | 14.2 | 99.1 |
| 2 (m) | H₃PO₄ | 2.27 | 45 | 30 | 24.3 | 88.9 |
| | | | | 60 | 27.1 | 88.4 |
| | | | | 90 | 28.0 | 85.6 |
| | | | | 120 | 28.5 | 82.8 |
| | | | | 150 | 28.9 | 81.2 |
| | | | | 180 | 29.2 | 79.1 |
| 3 (a) | ClSO₃H | 1.55 | 25 | 30 | 26.2 | 90.3 |
| | | | | 60 | 34.9 | 71.5 |
| | | | | 90 | 36.1 | 69.2 |
| | | | | 120 | 36.9 | 67.8 |
| 3 (b) | H₃PO₄ | 2.27 | 25 | 30 | 14.6 | 97.9 |
| | | | | 60 | 18.7 | 97.3 |
| | | | | 90 | 20.5 | 97.0 |
| | | | | 120 | 21.7 | 96.4 |
| | | | | 150 | 23.2 | 95.3 |
| | | | | 180 | 24.0 | 93.5 |
| 4 (a) | H₂SO₄ (96 wt-%) | 2.94 | 25 | 30 | 15.9 | 96.7 |
| | | | | 60 | 23.3 | 96.1 |
| | | | | 86 | 27.0 | 93.8 |
| | | | | 140 | 32.8 | 80.0 |
| | | | | 195 | 38.2 | 63.4 |
| | | | | 236 | 40.9 | 56.8 |
| 4 (b) | ClSO₃H | 3.5 | 25 | 8 | 10.0 | 98.1 |
| | | | | 15 | 20.6 | 95.6 |
| | | | | 30 | 28.4 | 83.5 |
| | | | | 45 | 35.2 | 69.1 |
| | | | | 60 | 36.5 | 65.6 |
| | | | | 75 | 37.5 | 63.5 |
| | | | | 90 | 38.8 | 61.2 |
| 5 (a) | HCl | | 25 | 5 | 5.2 | 94.5 |
| | | | | 10 | 6.1 | 95.2 |
| | | | | 15 | 8.0 | 95.9 |
| | | | | 30 | 10.2 | 96.6 |
| | | | | 60 | 13.2 | 96.8 |
| | | | | 120 | 15.3 | 91.5 |
| | | | | 180 | 16.7 | 94.7 |
| | | | | 240 | 17.8 | 96.4 |

## Claims

1. A catalyst composition for use in a hydrocarbon conversion process with the provision that the hydrocarbon conversion process is not cracking of polymers, which composition comprises
(a) an ionic liquid catalyst with an N-containing heterocyclic and/or aliphatic organic cation and an inorganic anion derived from metal halides or mixed metal halides, and
(b) one or more Brønsted Acids.

2. Catalyst composition of claim 1, wherein the cation of the ionic liquid catalyst is an N-aliphatic moiety with one or more alkyl or aryl groups.

3. Catalyst composition of claim 2, wherein the N-aliphatic moiety is an ammonium compound and/or an alkyl substituted pyridinium, piperidinium or quinolinium compound.

4. Catalyst composition of claim 1, wherein the anion of the ionic liquid is derived from a metal halide with strong Lewis acidic properties.

5. Catalyst composition of claim 1, wherein the ionic liquid catalyst is obtained by combining N-containing heterocyclic and/or N-containing aliphatic organic compounds with one or more metal halides in a molar ratio of between 1:3 and 1:0.5.

6. Catalyst composition of claim 1, wherein the metal halide is selected from AlCl₄⁻, AlBr₄⁻, GaCl₄⁻, AlₓCl₂ₓ₊₁⁻ , 1<x<2 and AlₓCl₂ₓBr⁻, 1<x<2.

7. Catalyst composition claim 1, where the Brønsted Acid is selected from ClSO₃H, FSO₃H, alkane sulphonic acids, fluorinated alkane sulphonic acids, carboxylic acids, fluorinated carboxylic acids and mineral acids.

8. A process for isomerisation of paraffinic hydrocarbons by contacting a feed stock comprising the paraffinic hydrocarbons with a composite catalyst according to any one of the preceding claims at process conditions being effective in the isomerisation of the paraffinic hydrocarbons.

9. Process of claim 8, wherein the composite catalyst is pretreated by heating at a temperature below 250°C.

10. Process of claim 8, wherein the process conditions comprise a pressure from 1 to 60 bar and a temperature from - 30°C to 150°C.
